(19) Europäisches Patentamt
European Patent Office
Office européen des brevets

(11) EP 3 035 055 B1

(12) # EUROPEAN PATENT SPECIFICATION

(45) Date of publication and mention of the grant of the patent:
**11.04.2018 Bulletin 2018/15**

(51) Int Cl.:
*G01N 33/543* (2006.01) *G01N 33/53* (2006.01)
*G01N 25/18* (2006.01)

(21) Application number: **15200962.7**

(22) Date of filing: **17.12.2015**

(54) **APTAMER-BASED BIOSENSING**

APTAMERBASIERTES BIOSENSING

BIODÉTECTION À BASE D'APTAMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2014 EP 14199245**

(43) Date of publication of application:
**22.06.2016 Bulletin 2016/25**

(73) Proprietors:
- **IMEC VZW**
  **3001 Leuven (BE)**
- **Universiteit Hasselt**
  **3500 Hasselt (BE)**

(72) Inventors:
- **PEETERS, Marloes**
  **3001 Leuven (BE)**
- **VAN GRINSVEN, Bart**
  **3001 Leuven (BE)**
- **DE CEUNINCK, Ward**
  **3001 Leuven (BE)**
- **WAGNER, Patrick**
  **3001 Leuven (BE)**

(74) Representative: **DenK iP**
**Leuvensesteenweg 203**
**3190 Boortmeerbeek (BE)**

(56) References cited:
- **M. PEETERS ET AL: "Heat-transfer-based detection of l-nicotine, histamine, and serotonin using molecularly imprinted polymers as biomimetic receptors", ANALYTICAL AND BIOANALYTICAL CHEMISTRY (SPRINGER), vol. 405, no. 20, 18 May 2013 (2013-05-18) , pages 6453-6460, XP055259045, New York ISSN: 1618-2642, DOI: 10.1007/s00216-013-7024-9**
- **M. PEETERS ET AL.: "Real-Time Monitoring of Aptamer Functionalization and Detection of Ara H1 by Electrochemical Impedance Spectroscopy and Dissipation-Mode Quartz Crystal Microbalance", JOURNAL OF BIOSENSORS & BIOELECTRONICS (OMICS PUBLISHING GROUP), vol. 05, no. 03, 21 July 2014 (2014-07-21) , pages 1-8, XP055259047, Hyderabad DOI: 10.4172/2155-6210.1000155**
- **JING ZHU ET AL: "Specific capture and temperature-mediated release of cells in an aptamer-based microfluidic device", LAB ON A CHIP (ROYAL SOCIETY OF CHEMISTRY), vol. 12, no. 18, 6 July 2012 (2012-07-06), page 3504, XP055259046, London ISSN: 1473-0197, DOI: 10.1039/c2lc40411g**
- **MARLOES PEETERS ET AL: "Label-free Protein Detection Based on the Heat-Transfer Method-A Case Study with the Peanut Allergen Ara h 1 and Aptamer-Based Synthetic Receptors", ACS APPLIED MATERIALS AND INTERFACES (AMERICAN CHEMICAL SOCIETY), vol. 7, no. 19, 8 April 2015 (2015-04-08), pages 10316-10323, XP055259040, Washington DC USA ISSN: 1944-8244, DOI: 10.1021/acsami.5b00994**

EP 3 035 055 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- GIDEON WACKERS ET AL: "Array Formatting of the Heat-Transfer Method (HTM) for the Detection of Small Organic Molecules by Molecularly Imprinted Polymers", SENSORS (MOLECULAR DIVERSITY PRESERVATION INTERNATIONAL), vol. 14, no. 6, 20 June 2014 (2014-06-20), pages 11016-11030, XP055259042, Basel DOI: 10.3390/s140611016
- BRAM GEERETS ET AL: "Optimizing the Thermal Read-Out Technique for MIP-Based Biomimetic Sensors: Towards Nanomolar Detection Limits", SENSORS ((MOLECULAR DIVERSITY PRESERVATION INTERNATIONAL)), vol. 13, no. 7, 16 July 2013 (2013-07-16), pages 9148-9159, XP055259043, Basel DOI: 10.3390/s130709148

SEQUENCE LISTING

<110> IMEC VZW Universiteit Hasselt

<120> Aptamer-based biosensing

<130> 15247IMr00EP

<160> 1

<170> BiSSAP 1.3

<210> 1
<211> 80
<212> DNA
<213> Artificial Sequence

<220>
<223> aptamer

<400> 1

tcgcacattc cgcttctacc gggggggtcg agctgagtgg atgcgaatct gtgggtgggc 60

cgtaagtccg tgtgtgcgaa 80

## Description

### Field of the invention

The invention relates to the field of aptamer sensors. More specifically it relates to a device and method for detecting a predetermined target analyte using an aptamer bioreceptor.

### Background of the invention

The detection and quantification of specific proteins in samples, such as biological, environmental or food samples, can be a complex and expensive process, e.g. when conventional microscopy and photometry methods are used. Biosensors can advantageously offer protein detection solutions that are fast, simple, cheap and portable. However, some biosensors known in the art may have the disadvantage of instability of the biological recognition component, e.g. an antibody or enzyme targeting the specific protein of interest. Such biosensor may typically comprise a receptor component and a transducer component, e.g. integrated in a single device. The receptor component, e.g. a protein recognition element, may use a biochemical mechanism for interacting with the target protein, while the transducer component may be adapted for generating an optical or electrical signal in response to the occurrence of such interactions.

Aptamers are a class of synthetic oligonucleic acid or peptide molecules that bind to a specific target, e.g. to specifically detect target molecules based on their three-dimensional configuration. For example, aptamers can consist of short strands of ribonucleic acid, desoxyribonucleic acid or a xenonucleic acid, or alternatively, aptamers can consist of short, variable peptide domains, e.g. short variable peptide domains attached on both ends to a protein scaffold. Aptamers can be obtained by selection from a large random sequence pool, e.g. by systematic evolution of ligands by an exponential enrichment process. In such process, selection of a suitable aptamer for detecting a predetermined template, e.g. an optimal aptamer for the template, may occur in vitro via a molecular process called Systematic Evolutions of Ligands by EXponential enrichment (SELEX) of libraries containing random sequences of oligonucleotides. With this procedure, it is possible to develop aptamers for a variety of targets, e.g. ranging from ions, to proteins, or even whole cells. The selected aptamers can furthermore be easily synthesized with high reproducibility and purity.

Aptamers can be used as in the receptor component of biosensors, e.g. as protein recognition elements, since such aptamers can selectively bind to a specific target due to a target-specific three dimensional structure. A substantial conformational change upon binding to the target protein induces a change in physical properties that then can be detected by the transducer component of the biosensor. Furthermore, proteins typically have a complex structure that allows a binding to aptamers by various mechanisms, e.g. electrostatic interactions, hydrogen bonds and/or complementary of three-dimensional structure.

This use of aptamers offers various advantages, such as a fast and easy to perform detection process, and a good sensitivity and selectivity. Because aptamers can be relatively small in size, e.g. less than 100 nucleotides, compared to other bioreceptors, e.g. relative to antibodies and enzymes, a high density of bioreceptors can be achieved in a biosensor. Another particularly advantageous property of aptamers as protein recognition element is their good stability and a good reproducibility of the target interaction mechanism. Furthermore, the aptamer receptor component of a biosensor can be advantageously regenerated.

Aptamer-based biosensor systems, also referred to as aptasensors, for detecting templates with good specificity and selectivity are known in the art. An aptasensor can furthermore be easily adapted for detecting a specific protein, but also for the detection of other analytes of interest, such as whole cells or small molecules in complex samples.

Aptamer biosensors using electrochemical transduction components are known, e.g. based on voltammetry, potentiometry, amperometry or impedance measurements. Furthermore, optical transduction methods, such as surface plasmon resonance, evanescent wave spectroscopy, fluorescence anisotropy detection, luminescence detection and colorimetric assays, are also known in the art. Other known transduction mechanisms comprise capillary electrophoresis and microgravimetric sensing. Although such techniques can achieve a high specificity and selectivity, they may not be easily integrated into a compact and portable biosensor setup, may require an expensive set up and/or may require long measurement times.

For example, an aptamer-based impedance spectroscopy sensor platform was disclosed by Peeters et al. in J. Biosens. Bioelectron. 5 (DOI: 10.4172/2155-6210.1000155). Such sensor platform can advantageously provide a low cost protein measurement system, e.g. suitable for measuring peanut allergen Ara h 1 concentrations in buffer solutions in the lower nanomolar range. While such electrochemical transduction measurements allow some degree of system integration and miniaturization, the analysis can still be complicated and may require a bulky impedance analyzer. Even though it may be feasible to integrate an impedance analyzer in a small format device, e.g. a pocket-sized device, such impedance analyzer would still be relatively complicated, e.g. may require technologically advanced electronic components. Furthermore, the interpretation of results obtained by such impedance analysis may require substantial experience and may rely on subjective interpretation.

The document "Heat-transfer-based detection of 1-nicotine, histamine, and serotonin using molecularly imprinted polymers as biomimetic receptors," in Analytical and bioanalytical chemistry, vol. 405, no. 20, pp. 6453-6460, by Peeters et al, discloses a heat transfer method (HTM) for the detection of small molecules with molecularly imprinted polymer (MIP)-type receptors, based on a change in heat-transfer resistance imposed upon binding of target molecules to the MIP microcavities.

The document "Optimizing the thermal read-out technique for MIP-based biomimetic sensors: towards nanomolar detection limits," in Sensors, vol. 13, no. 7, pp. 9148-9159, by Geerets et al, also discloses a HTM for the detection of small molecules with MIP-type receptors.

The document "Specific capture and temperature-mediated release of cells in an aptamer-based microfluidic device," in Lab on a chip, vol. 12, no. 18, p. 3504, by Zhu et al, discloses a method that allows easy release and collection of live cells from affinity surfaces for subsequent analysis and detection, using a microfluidic device. This prior-art approach enables a non-destructive, temperature-mediated release and retrieval of cells, in which specific cell capture is achieved using surface-immobilized aptamers.

The document "Array formatting of the heat-transfer method (HTM) for the detection of small organic molecules by molecularly imprinted polymers," in Sensors, vol. 14, no. 6, pp. 11016-11030, by Wackers et al, discloses a MIP-based biomimetic sensor array for the detection of small organic molecules via the heat transfer method, using a flow-through sensor cell.

**Summary of the invention**

It is an object of embodiments of the present invention to provide good specificity and selectivity in a compact aptamer-based detection system.

The above objective is accomplished by a method and device according to the present invention.

It is an advantage of embodiments of the present invention that a predetermined protein of interest can be detected. It is furthermore an advantage of embodiments of the present invention that detection of a predetermined analyte of interest can be achieved in a complex sample, e.g. a predetermined protein, whole cell or small molecule can be detected in accordance with embodiments of the present invention using substantially the same technology platform.

It is an advantage of embodiments of the present invention that detection can be performed without being laborious, expensive or requiring complex techniques.

It is an advantage of embodiments of the present invention that detection can surprisingly be applied directly for the screening of trace allergens in food samples, e.g. without requiring complex sample preparation or chemical treatment steps.

It is an advantage of embodiments of the present invention that aptamer protein detection can be achieved with good specificity and selectivity in a compact biosensor setup, e.g. in a portable biosensor.

It is an advantage of embodiments of the present invention that protein biosensors are provided that have a highly chemically stable protein receptor component, e.g. a chemically stable protein detection element. For example, the protein biosensor according to embodiments may have a receptor component that is resilient to degradation by endogenous biological agents in cell lysates and/or serum, e.g. to endogenous ribonucleases.

It is an advantage of embodiments of the present invention that protein biosensors are provided that can be reused and/or regenerated.

It is an advantage of embodiments of the present invention that transduction by heat-transfer in accordance with embodiments can enable protein detection in a fast and low-cost manner. Furthermore, such heat-transfer transduction can fully integrated in a compact biosensor device, e.g. in an integrated circuit device.

In a first aspect, the present invention relates to a biosensor device for detecting a predetermined target analyte. The biosensor device comprises a substrate comprising an aptamer bioreceptor exposed at a functionalized surface thereof, the aptamer bioreceptor being adapted for specifically binding to the predetermined target analyte; a heat source for heating said substrate via a back surface thereof, the back surface being opposite of said functionalized surface and directed toward the heat source; a first temperature sensing element for sensing a temperature at the side of the back surface of the substrate and a second temperature sensing element for sensing a temperature at the side of the functionalized surface of said substrate; and a signal processing unit adapted for calculating at least one heat transfer resistivity value based on temperature values obtained from the first temperature sensing element and the second temperature sensing element and the heating power generated by the heat source.

In a biosensor device according to embodiments of the present invention, the aptamer bioreceptor may be adapted for specifically binding to a predetermined target protein.

In a biosensor device according to embodiments of the present invention, the aptamer bioreceptor may comprise a strand of synthetic oligonucleic acid or peptide domains.

In a biosensor device according to embodiments of the present invention, the substrate may comprise a semiconductor substrate, a metal deposited on the surface of the semiconductor substrate, and a self-assembled monolayer deposited on the metal. The aptamer bioreceptor may be chemically coupled to the self-assembled monolayer (22) such as to form said functionalized surface.

In a biosensor device according to embodiments of the present invention, the aptamer bioreceptor may comprise a spacer segment, e.g. a carbon spacer segment, for spacing the aptamer bioreceptor away from the substrate.

In a biosensor device according to embodiments of the present invention, the substrate may furthermore comprise a blocking layer to reduce non-specific binding.

In a biosensor device according to embodiments of the present invention, the heat source may comprise a solid body heat sink and a heating element for converting an input power, e.g. an input electric power, to a caloric power.

In a biosensor device according to embodiments of the present invention, the first temperature sensing element may be configured to measure a temperature of the solid body heat sink.

In a biosensor device according to embodiments of the present invention, the signal processing unit may comprise a proportional-integral derivative controller for actively steering the temperature of the heat source, the proportional-integral derivative controller may be adapted for receiving a signal representative of the temperature of the solid body heat sink as input and may be adapted for outputting a signal to control the power of the heat source.

In a biosensor device according to embodiments of the present invention, the first temperature sensing element and/or the second temperature sensing element may comprise at least one thermocouple.

In a biosensor device according to embodiments of the present invention, the substrate may be substantially horizontally arranged such that the functionalized surface is directed downward.

A biosensor device according to embodiments of the present invention may furthermore comprise a flow cell for bringing said functionalized surface of the substrate into contact with a liquid sample, and said flow cell may comprise a fluid compartment for exposing the functionalized surface of the substrate to the liquid sample.

In a biosensor device according to embodiments of the present invention, the flow cell may comprises a pumping and/or valve system for transferring fluid from and to the fluid compartment.

In a biosensor device according to embodiments of the present invention, the second temperature sensing element may be positioned in said fluid compartment.

In a second aspect, the present invention relates to a method for detecting a predetermined target analyte, the method comprising: obtaining a substrate having a functionalized surface, the substrate comprising an aptamer bioreceptor exposed at said functionalized surface, wherein said aptamer bioreceptor is adapted for specifically binding to the predetermined target analyte; providing a heating power for heating said substrate at a back surface thereof, said back surface being opposite of said functionalized surface; sensing at least a temperature at a first side of the substrate corresponding to said back surface and at a second side of the substrate corresponding to said functionalized surface; and calculating at least one heat transfer resistivity value based on the temperature values obtained at the first side and the second side and the heating power for deriving a characteristic of the predetermined target analyte from said heat transfer resistivity value.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

## Brief description of the drawings

FIG. 1 schematically shows a biosensor according to embodiments of the present invention.

FIG. 2 shows a biosensor device according to embodiments of the present invention.

FIG. 3 shows a relative increase of $R_{th}$ over 4000 s during an exemplary functionalization reaction in a biosensor device according to embodiments of the present invention.

FIG. 4 shows an exemplary exponential curve fit to the functionalization procedure of a biosensor device according to embodiments of the present invention.

FIG. 5 shows exemplary absolute values of $R_{th}$ after addition of Ara h 1 to a fully functionalized substrate in accordance with embodiments of the present invention.

FIG. 6 shows exemplary relative values of $R_{th}$ after addition of Ara h 1 to a fully functionalized substrate in accordance with embodiments of the present invention.

FIG. 7 shows exemplary results of the analysis of a peanut extract in accordance with embodiments of the present invention.

FIG. 8 shows exemplary results of the analysis of a peanut extract in accordance with a method known in the prior art.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

**Detailed description of illustrative embodiments**

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in embodiments of the present invention reference is made to heat transfer resistivity $R_{th}$, reference is made to the ratio $R_{th}=\Delta T/P$ of the temperature difference $\Delta T$ of the temperature at each side of a substrate to be tested to the input power P.

In a first aspect, the present invention relates to a biosensor device for specifically detecting a predetermined target analyte, e.g. for the detection, characterization and/or quantification of the predetermined target analyte. By way of illustration, embodiments of the present invention not limited thereto, a schematic overview of standard and optional components is shown in FIG. 1.

The biosensor device 10 for detecting a predetermined target analyte, such as a predetermined target protein, in accordance with embodiments of the present invention comprises a substrate 13. This substrate 13 comprises an aptamer bioreceptor exposed at a functionalized surface of the substrate. This aptamer bioreceptor is specifically adapted for binding to the predetermined target analyte, for example, the aptamer bioreceptor 21 may be adapted for specifically binding to a predetermined target protein.

Furthermore, it is an advantage of emodiments of the present invention that the aptamer bioreceptor does not need to be forced into a substantially linear conformation, e.g. by applying a neutralizer, such as an almost complementary nucleic acid strand. Thus, contrary to methods which may require a neutralizer-displacement method, the conformation of the aptamer bioreceptor 21 may be free, e.g. may be only limited by the binding of the aptamer bioreceptor to the surface of the substrate and/or to the target analyte when present.

The substrate may be advantageously obtained from a substantially flat plate, e.g. the substrate does have cavities formed therein to enable the selective binding of the target analyte. Thus, even though the substrate may have at least an aptamer bioreceptor attached thereto, it may be substantially flat in the sense that it does not have shallow or deep surface cavities formed therein. Therefore, it is an advantage of embodiments of the present invention that no mechanical processing, such as imprinting or laser ablation milling, are required to make the substrate specifically suitable for detection of the predetermined analyte. The substrate 13 may be arranged such as to expose the substrate to a heating element 11.

The biosensor device 10 for detecting a predetermined target analyte in accordance with embodiments of the present invention comprises a heat source 11, e.g. an adjustable and controllable heat source, for heating the substrate 13 via a back surface of the substrate. The back surface of the substrate is opposite of the functionalized surface, and is directed toward the heat source 11. The heat source 11 may for example comprise a block of solid material, e.g. a heat sink, and a heating element such as a power resistor for converting an input power to a caloric power. In principle any type of heating element may be used. The heat source according to embodiments may be adapted, e.g. by its position and orientation with respect to the substrate, such that a temperature gradient is created over the substrate. Heating elements thus may be used that provide a heating source at one side of the substrate, which transfers through the substrate, and then may go into a fluid positioned at the opposite side of the substrate, e.g. opposite to the heat source in relation to the substrate. Furthermore, it is an advantage of embodiments of the present invention that such fluid, e.g. that may contain the sample to be tested, does not need to conform to predetermined electrical conductivity requirements, as may be the case in related methods or devices known in the art, but can be any suitable fluid, e.g. an alcohol such as ethanol or an oil.

The biosensor device 10 also comprises a first temperature sensing element 14 for sensing a temperature at the back side where the substrate 13 is exposed to the heat source 11, e.g. at a side of the substrate corresponding to the back surface of the substrate, and a second temperature sensing element 15 for sensing a temperature at the functionalized side of the substrate 13, e.g. at a side of the substrate corresponding to the functionalized surface. The temperature sensing elements 14, 15 may be any type of temperature sensing element, e.g. may comprise a thermocouple, a thermopile, a thermistor, a thermometer, a pyroelectric element, a resistance temperature detector and/or an infrared detector. The device 10 may also comprise more than two sensing elements, such as for example an array of temperature sensing elements, although for operating embodiments of the invention, two temperature sensing elements arranged on opposite sides of the substrate 13 may be sufficient.

The biosensor device 10 furthermore comprises a signal processing unit 16, e.g. a processing means such as a processor. This signal processing unit 16 is adapted for, e.g. programmed for, calculating at least one heat transfer resistivity value based on temperature values obtained from the first temperature sensing element 14 and the second temperature sensing element 15 and the heating power generated by the heating element 11. The signal processing unit 16 may furthermore be adapted for deriving a characteristic of the target analyte from the heat transfer resistivity value, for example an indicator of the presence of the target analyte or a concentration estimate of the target analyte. In certain advantageous embodiments, the signal processing unit 16 may be adapted for determining a heat transfer resistivity as function of temperature. The signal processing unit 16 may furthermore be adapted for filtering the data, e.g. in order to improve a signal to noise ratio. The processing means 16 furthermore may be adapted for deriving from the heat transfer resistivity values a characteristic of the target analyte. The signal processing unit 16 may be adapted for taking experimental conditions into account. The signal processing unit 16 may be programmed for performing the above in an automated way. Such signal processing unit 16 may comprise a software based processor and/or a hardware based processor. It may for example make use of a predetermined algorithm, a look up table or a neural network for performing the processing. However, it shall be clear to the person skilled in the art that calculating the heat transfer resistivity value may relate to calculating a value representative of the heat transfer resistivity, e.g. a value being related thereto by a functional relationship, e.g. a monotonic functional relation. For example, a value representative of the temperature sensed by the second temperature sensing element 15 may be directly representative of the heat transfer resistivity, e.g. under tightly controlled conditions. The value representative of the heat transfer resistivity may also be a non-scalar value, e.g. a time series of temperatures obtained from the first and second temperature sensing element. For example, a short heat pulse may be applied via the heat source, and the evolution of both temperatures as function of time may be obtained a characteristic of the heat resisitivity.

It is an advantage of embodiments of the present invention that a heat-transfer method (HTM) can be used for transduction in an aptasensor device, thus offering detection of an analyte of interest in a fast and low-cost manner. It is an advantage of embodiments of the present invention that HTM transduction can be performed in a fully integrated biosensor device. Embodiments of the present invention have the advantage of being simple to implement and use, without requiring complex hardware.

FIG. 2 shows an exemplary biosensor device 10 according to embodiments of the present invention. The device comprises a substrate 13 and a heat source 11 for heating the substrate 13 via a back surface of the substrate.

The heat source 11 may for example comprise a block of solid material 19 and a heating element such as a power resistor 20 for converting an input power to a caloric power. The heat source 11 may comprise a heat sink, for example a solid body heat sink 19, e.g. a copper block. The substrate 13 may thus be pressed mechanically onto the solid body heat sink 19. This block served as a heat sink and thermal transport may for example occur through the substrate to a liquid 24 on the opposite side of the substrate 13.

The substrate may for example be a semiconductor substrate having a metal deposited on its surface. For example, the substrate may comprise a metal at the functionalized surface that has good thermal conductivity and good chemical stability, such as gold. For example, a layer of chromium may be deposited onto a doped silicon substrate, onto which a layer of gold is deposited. The functionalized surface may be a gold surface activated by ultra-violet ozone treatment. The functionalized surface may be a bio-functionalized surface, e.g. the substrate 13 may comprise a self-assembled monolayer (SAM) 22 at the functionalized surface side, e.g. a self-assembled monolayer of mercaptoundecanoic acid (MUA) on gold.

It is to be noted that the substrate may be a semiconductor material, e.g. doped silicon, or a metal substrate, e.g. aluminium, or any suitable solid material having good thermal conductivity and being directly, or at least indirectly via intermediate layers or treatments, suitable for functionalization with bioreceptors in accordance with embodiments of the present invention. For example, the substrate may comprise sapphire material, e.g. synthetic sapphire. It is an advantage of embodiments of the present invention that the substrate material is not required to have good electrical conductivity.

The substrate 13 comprises an aptamer bioreceptor 21 exposed at a functionalized surface of the substrate. This aptamer bioreceptor may be specifically adapted for binding to the predetermined target analyte. For example, the aptamer bioreceptor may comprise a synthetic oligonucleic acid or peptide molecule adapted for binding to a specific target. The aptamer bioreceptor may comprise a strand of ribonucleic acid, desoxyribonucleic acid or xenonucleic acid. Alternatively, the aptamer bioreceptor may comprise peptide domains, for example peptide domains attached to a protein scaffold. The aptamer bioreceptor may be adapted for binding to a predetermined target analyte such as a predetermined protein. The aptamer bioreceptor 21 may be covalently coupled to the substrate 13, e.g. to the self-assembled monolayer 22, e.g. by an amide bond obtained by a carboxyl activating agent acting on an amino-terminated end of the aptamer bioreceptor when in aqueous solution. Furthermore, the aptamer bioreceptor may comprise a spacer segment, e.g. a carbon spacer segment, e.g. a linear chain having N carbon atoms, where N is in the range of 1 to 25, e.g. 6, or even longer, e.g. any number of carbon atoms, as will be clear to the person skilled in the art as being suitable as spacer, e.g. 30 carbon atoms, 50 carbon atoms, 100 carbon atoms or even more. Furthermore, although a linear chain of carbon atoms is a convenient choice, the person skilled in the art will appreciate that any spacer could be used, e.g. comprising not only carbon atoms as structural scaffolding elements, or even comprising only other atoms. The spacer segment may be at the end of the aptamer bioreceptor 21 that is attached to the substrate 13.

The substrate 13 may furthermore comprise a blocking layer 23 to reduce non-specific binding, e.g. to prevent non-specific binding of molecules, ions or biological entities other than the predetermined analyte to the functionalized surface of the substrate. For example, bovine serum albumin (BSA), e.g. attached to the self-assembled monolayer, e.g. to a MUA self-assembled monolayer, may substantially cover the functionalized surface of the substrate 13 where left exposed by the aptamer bioreceptor. For example, gold surface of the substrate that did not react with the aptamer bioreceptor may be blocked by BSA.

The device may also comprise a flow cell 25 for bringing the functionalized surface of the substrate 13 into contact with a liquid sample to test 24. Furthermore, the substrate 13 may be advantageously arranged substantially horizontally, e.g. horizontally, with the functionalized surface directed downward. Thus, heavy components in the liquid sample may be prevented from sinking to the functionalized surface and thereby reducing non-specific binding. The biosensor device 10 may for example be adapted for being mechanically supported by a support structure, e.g. a level surface, such that the substrate 13 is oriented horizontally with the functionalized surface directed downward when the device is in operation, e.g. downward with respect to the ambient gravitational field. Furthermore, a horizontal arrangement of the substrate with the functionalized surface directed downward may also advantageously improve the temperature profile, e.g. may provide a layered temperature profile, for example by preventing convective heat transport. For example, when the functionalized surface is directed upward, convection may contribute more to the heat transport, e.g. when an aqueous medium is used. Therefore, lower apparent heat resistivity values may be observed when such arrangement is used. Even though the heat transfer method for quantifying the specific aptamer binding would still be valid, higher values for the downward directed arrangement, and therefore higher apparent heat resistivity values, may advantageously provide better sensitivity and accuracy.

According to some embodiments of the present invention, the biosensor device 10 may comprise a fluid compartment 28 for exposing the functionalized surface of the substrate 13 to a fluid 24. The second temperature sensing element 15 may be positioned in the fluid compartment 28. The fluid may be used for introducing the target analyte to be analyzed, e.g. in an aqueous solution or in a fluid medium. The biosensor device 10 may comprise a flow cell 25 comprising the fluid compartment 28 and furthermore comprising a pumping and/or valve system for transferring fluid from and to the fluid compartment 28. According to particular embodiments, the flow cell may comprise a syringe system coupled to a Perspex flow cell with a suitable inner volume, e.g. around 110 μl for a simple test device setup. In some embodiments, the fluid compartment may be sealed, e.g. onto the substrate and/or the heat sink, by an O-ring 29.

The first temperature sensing element 14 for sensing a temperature at the back side where the substrate 13 is exposed to the heat source 11 may for example comprise a thermocouple configured to measure the temperature $T_1$ of the heat sink 19, e.g. the copper block. The signal processing unit 16 may comprise a PID controller 18 for actively steering the temperature of the heat source 11, e.g. of the heat sink 19. This PID controller may receive a signal representative of the temperature sensed by the first temperature sensing element 14 as input and may output a signal to control the power of the heat source 11, e.g. via a power resistor 20. Thus, the signal processing unit 16 may comprise a controller adapted for controlling the heat source 11 and for controlling the temperature sensing elements such as to obtain input power and temperature values. Such values may be obtained for different input powers, or-corresponding therewith-for different temperatures as sensed with the first temperature sensing element.

The second temperature sensing element 15 for sensing a temperature at the functionalized side of the substrate 13 may for example comprise a thermocouple configured to measure a second temperature $T_2$ at a distance from the substrate. The distance to the substrate may preferably be as low a achievable, e.g. less than 5 mm, for example less than 2 mm, e.g. 1.7 mm, or even less, for example 1 mm or less. A short distance to the substrate, e.g. to the functionalized surface, advantageously provides an improved relative effect, e.g. the contribution of the heat resistivity of the medium separating the substrate from the second temperature sensing element is advantageously reduced by shorter distances. The second temperature sensing element 15 may be arranged in a fluid container for containing the liquid sample to test 24, e.g. in the fluid compartment 28.

This signal processing unit 16 is adapted for, e.g. programmed for, calculating at least one heat transfer resistivity value based on temperature values obtained from the first temperature sensing element 14 and the second temperature sensing element 15 and the heating power generated by the heating element 11. The thermal resistance $R_{th} = \frac{T_1 - T_2}{P}$, may for example be obtained by dividing the temperature difference $T_1$ - $T_2$ by the input power P of the power resistor 20 that is required to keep the heat sink 19 at a constant temperature.

When the analyte of interest binds to the aptamer bioreceptor, the properties at the solid-liquid interface are affected, e.g. due to a configuration change induced by the binding. This causes a change in heat conduction through the functionalized surface, which is then registered by the signal processing unit 16. For example, the biosensor device 10 may be adapted for determining a protein concentration of a specific protein with this technique. For example, the signal processing unit 16 may correlate the thermal resistance via a predetermined dose-response curve to a concentration of the analyte of interest. It is an advantage of embodiments of the present invention that an analyte of interest can be detected and/or quantified in a sample using remarkably simple methods and measuring equipment, which are particularly suitable for integration in sensor platforms, e.g. in a portable sensor system, such as a handheld sensor.

In a biosensor device 10 according to embodiments of the present invention, the signal processing unit 16 may be adapted for outputting a characteristic of the predetermined target analyte based on the at least one heat transfer resistivity value. The heat source 11 may be controlled by a power resistor providing an input power. The first temperature sensing element and/or the second temperature sensing element may be a thermocouple. The biosensor device may comprise a controller for controlling the heating element and for controlling the temperature sensing elements for obtaining input power and temperature values for different temperatures as sensed with the first temperature sensing element.

The biosensor device 10 according to embodiments may also comprise electrodes for measuring an impedance, or may be adapted for measuring a fluorescence signal emitted from the functionalized surface, as the heat transfer method of measurement can be easily combined with other measurement techniques, e.g. for cross-checking. Further optional features and advantages may be as described in the example below. In another example, the biosensor device 10 according to embodiments may be combined with a microbalance for performing QCM measurements.

In a second aspect, the present invention relates to a method for characterising, quantifying and/or detecting a predetermined target analyte. According to certain embodiments of the present invention, the method comprises obtaining a substrate 13 having a functionalized surface. The substrate comprises an aptamer bioreceptor 21 exposed at the functionalized surface, in which this aptamer bioreceptor is adapted for specifically binding to the predetermined target analyte.

The method further comprises providing a heating power using a power at a back side of the substrate. Thus, a heating power may be provided for heating the substrate 13 at a back surface thereof, in which the back surface is opposite of the functionalized surface. This providing of the heating power may use a power, e.g. an electrical power converted into caloric energy. This results in a temperature gradient over the substrate and thus over the target analyte to be characterised and/or detected, when bound to the aptamer bioreceptor.

The method also comprises sensing at least a temperature at a first side of the substrate corresponding to the back surface of the substrate. The method also comprises second at least a temperature at a second side of the substrate corresponding to the functionalized surface of the substrate.

From these measurements and the power used for heating the substrate, e.g. the power consumed by a heating element, according to certain embodiments of the present invention, at least one heat transfer resistivity value is calculated for detecting the predetermined target analyte and/or for deriving a characteristic of the predetermined target analyte from the heat transfer resistivity value. In some embodiments, calculating at least one heat transfer resistivity value comprises determining the heat transfer resistivity as function of temperature, e.g. determining different heat transfer resistivity values at different temperatures. The calculating may furthermore include applying a filter for improving the signal to noise ratio. The temperature used as reference can in principle be chosen and may for example be the temperature sensed with the first temperature sensing element. In operation, the first temperature is preferably higher than an ambient temperature, e.g. a temperature of the environment, e.g. than room temperature.

In certain embodiments according to the present invention, the substrate may be brought into contact with a fluid, e.g. a fluid to be tested for the presence or concentration of the predetermined target analyte. Thus, temperature sensing on the functionalized side may occur in the fluid.

Further optional steps of a method according to embodiments of the present invention may be expressed as the functionality of components described in the first aspect, or may correspond with features as described in the example below. Advantageously, the method may be used with a device according to an embodiment as described in the first aspect, although embodiments of the present invention are not limited thereto.

Other aspects of the present invention may relate to the use of a device according to the first aspect of the present invention for the detection of peanut allergen Ara h 1 in a food sample, e.g. for the estimation of a concentration of peanut allergen Ara h 1 in a food sample. The invention may also relate to the use of the device according to the first aspect of the present invention for the detection and/or quantification of an analyte of interest in a food sample, a soil or water sample, or in a tissue or blood sample.

By way of illustration, embodiments of the present invention are illustrated by the following examples. These examples relate to a particular biosensor and to experimental results obtained therewith, and illustrate features and advantages of embodiments according to the present invention. However, it should be understood that the invention is not in any way limited by the specific details provided by these examples. It should be kept in mind that the following examples should not be construed as limiting the inventive concept to any particular physical configuration or features. Although illustrated in the context of peanut allergen Ara h 1 detection, it should be appreciated that the target protein could take the form of any protein or other analyte that is detectable by an aptamer, e.g. by a suitable aptamer obtainable via an exponential selection process or any alternative aptamer selection process. The examples provided hereinbelow demonstrate that peanut allergen Ara h 1 can be detected in accordance with embodiments of the present invention in buffer solutions, and also in complicated matrices.

A surprisingly high increase in heat-transfer resistance upon binding of the target to the aptamer can be achieved in accordance with embodiments of the present invention. Furthermore, this method can be easily applied directly on food samples, e.g. without requiring additional processing or sample preparation steps, except insofar providing the sample in a solution or other liquid form.

In this example, thermal detection measurements of peanut allergen Ara h 1 in buffer solutions were performed with an aptamer-based sensor platform according to embodiments of the present invention. This exemplary system was selected because the recognition between the aptamer and Ara h 1 has already been thoroughly studied in the technical field. Furthermore, since the peanut allergen Ara h 1 is responsible for a significant fraction of food-related anaphylactic shock incidents, this example also illustrates a particularly relevant application for the food industry. The results presented hereinbelow achievable detection limits in the low nanomolar regime. To further illustrate a practical exemplary application, spiked Ara h 1 concentrations were studied in a food matrix enriched with peanut butter. These results demonstrate that embodiments of the present invention have applicability beyond achieving good detection sensitivity and specificity in controlled buffer solutions. Nevertheless, a sensor platform according to embodiments of the present invention can also detect other proteins for which corresponding aptamers exist, e.g. thereby requiring only minor modifications, e.g. with respect to the setup disclosed by this example, that are well-known to the person skilled in the art.

For this example, 11-mercapto-undecanoic acid (MUA) and bovine serum albumin (Mr about 66.5 kDa, BSA) were obtained from Sigma Aldrich (Steinheim, Germany) and 1-ethyl-3-(3-dimethylaminopropyl) (EDC) was obtained from Thermo Scientific (Aalst, Belgium). The peanut allergen Ara h 1 and its competitor Ara h 2 were obtained from INDOOR technologies (Cardiff, Wales) and used as received. Binding of Ara h 2 to the aptamer at hand may be considered negligible, as has been documented in the art. Compounds used for buffer preparation of 2-(N-morpholino)ethanesulfonic acid (MES) buffer, tris(hydroxyamino)methane-glycine-potassium (TGK) buffer and phosphate buffered saline (PBS) buffer were purchased from Sigma Aldrich (Steinheim, Germany) and Fisher Scientific (Landsmeer, The Netherlands). Analytical grade ethanol (anhydrous, 99.9%) was obtained from VWR (Leuven, Belgium). The aptamer sequence for Ara h 1 detection used for this example is known in the art, as disclosed by Tran et al. in Biosens. Bioelectron. 43, p. 245-251, and was ordered from IDT Technologies (Leuven, Belgium).

The aptamer sequence corresponded to the following 80 base pairs sequence known in the art:

5'TCGCACATTCCGCTTCTACCGGGGGGGTCGAGCTGAGTGGATGCGAATCTGTGG GTGGGCCGTAAGTCCGTGTGTGCGAA 3'

In the present example, the Ara h 1 detection aptamer was further processed by modifying the 5' end with an amino group and a C6 carbon spacer. The structural conformation of the aptamer at certain temperatures can be found in Peeters et al., J. Biosens. Bioelectron. 5 (DOI: 10.4172/2155-6210.1000155). The carbon spacer may allow the aptamer to bind freely near a solid surface, e.g. by reduction of steric hindrance.

The samples for the present example consisted of gold substrates of 1x1 $cm^2$ that were prepared as follows. A 20 nm adhesive layer of chromium was evaporated onto doped silicon, followed by a 80 nm layer of gold at 5 x $10^{-5}$ Pa. These were treated with a Digital PSD series UV-ozone system from Novoscan (Nurnberg, Germany) for one hour in order to activate the surface. Subsequently, they were briefly exposed to a cold "piranha" solution ($H_2O_2$:$H_2SO_4$ 1:3), rinsed with ethanol, and then incubated for 48 h with a MUA solution in ethanol (1 mM) under nitrogen atmosphere. After cleaning the samples, the amino-terminated Ara h 1 aptamers were attached via EDC coupling in MES buffer of pH 6. This process was monitored in-real time by probing the thermal resistance at the solid-liquid interface. In order to reduce non-specific binding, the unreacted gold surface was blocked by immersing the substrates overnight into a BSA solution (50 nM in PBS). After stabilizing in TGK buffer, various solutions of Ara h 1 in TGK buffer, at concentrations of respectively 5, 10, 15, 25 and 50 nM, were added to the set up. To address the specificity and selectivity, reference experiments were performed with a gold substrate with only the SAM and without the presence of the aptamer, and by exposing the fully functionalized aptasensor to the competitor molecule Ara h 2.

Measurements were performed in a matrix enriched with peanut butter. To obtain these samples, 50 mg of peanut butter (Calvé, The Netherlands) was first molten and then dissolved into 200 ml of TGK butter. After filtration, the resulting fluid was split into two aliquots, of which one was unaltered and the other spiked with 100 nM of Ara h 1. The amount of Ara h 1 in the buffer diluted extract, which was in the order of about 1 to 5 nM, can be estimated according to the procedure described in Pomés et al., Clin. Exp. All. 36, p. 824-830.

Thermal resistance measurements were performed, in accordance with embodiments of the present invention, using a system design as depicted in FIG. 1. To this system, a Perspex flow cell with an inner volume of 110 $\mu$l was coupled. The functionalized gold samples were mounted horizontally in the set up in order to prevent heavy components from sinking to the surface and thereby reducing non-specific binding.

The gold substrate, functionalized with Ara h 1 aptamer, was pressed mechanically onto the copper block. This block served as a heat sink and thermal transport occurred through the gold sample to the liquid. The temperature $T_1$ of the copper, was measured by a thermocouple and actively steered through a PID controller, having parameters P = 8, I = 1 and D = 0, which in turn was connected to a power resistor. During the measurements, this temperature was kept constant at 37.00 °C, e.g. in order to mimic body temperature. At 1.7 mm above the sample surface in the liquid, a second temperature $T_2$ was measured with a second thermocouple. The thermal resistance $R_{th} = \frac{T_1 - T_2}{P}$, e.g. expressed in units of °C/W, can then be obtained by dividing the temperature difference $T_1$ - $T_2$, e.g. expressed in degrees Celsius, by the input power P, e.g. expressed in Watt, that is required to keep the copper block at a constant temperature. During the measurement, the ambient temperature in this example remained constant at 19.0 °C. When Ara h 1 binds to the aptamer, the properties at the solid-liquid interface are affected, e.g. due to a configuration change induced by the binding. The size of this effect increases when more Ara h 1 binds to the aptamers.

For aptamer functionalization, a baseline was established by immersing the substrate into MES buffer. Then, a solution containing MES buffer with aptamer (0.1 $\mu$M) and EDC (400 mM) was added. In the graph shown in FIG. 3, averages over 1000 consecutive measurement points each are shown, as collected over consecutive time intervals of 1000. The graph shows the relative increase in $R_{th}$ as function of time, as indicated by Average 1, Average 2, Average 3 and Average 4. Each point corresponds to the average taken over 1000 points in a timeframe of 1000 s, e.g. a single point collected per second. After 4000 s, the reaction is complete and the $R_{th}$ has increased to 107.9 $\pm$ 0.8 °C/W. Between 3000 and 4000 s, $R_{th}$ does not substantially change anymore, e.g. indicating that the reaction is complete. This corresponds to a normal reaction time of approximately 2 h, as known in the art.

FIG. 4 shows an exponential curve fitted to the functionalization procedure in order to get an estimate for the time constant ($\tau$). This $\tau$ is approximately 1.2 h, what one would expect from the EDC coupling, e.g. about 2 h. The equation fit follows the equation y = Al*exp(-x/t1) +y0. The corresponding values for the equation are given in the table below.

| Adj. R-square | 0,94212 | | |
|---|---|---|---|
| | Re | Value | Standard Error |
| B | y0 | 114,8110 | 11,02046 |
| B | A1 | -18,51274 | 9,07333 |
| B | t1 | 4,6426 | 5,05914 |

FIG. 5 shows absolute values of $R_{th}$ after addition of Ara h 1 to a fully functionalized substrate, after a stabilizing period of about 30 min. Injections were done manually in volumes of 1 ml, approximately every 20 to 30 min. FIG. 6 shows the corresponding relative value of $R_{th}$, along with reference results in which only the SAM layer blocked with BSA is present. Note that the BSA layer is present in order to avoid a large amount of non-specific absorption to the surface. As can be seen from FIG. 6, the lower limit of detection, corresponding to a confidence level of 3 standard deviations, can be estimated from the allometric curve fit y = a xb, with a = 100, b = 0.027 and goodness of fit $R^2$ = 0.96. The standard deviation on the lowest point is 0.5, therefore the limit of detection would be around 101.5. This corresponds to a limit of detection (LOD) of about 3 nM and a limit of quantification of about 4 nM, corresponding to the concentration at which the signal equals 5 times the standard deviation. This is comparable to what can be achieved with an aptamer biosensor using impedance spectroscopy transduction, and slightly higher than what can be obtained with natural antibody biosensor, e.g. less than 1 nM.

For the exemplary results in which Ara h 1 was measured in a peanut butter enriched matrix, first a baseline was established in TGK buffer after which the peanut extract was added (50 mg in 200 ml TGK), followed by the extract spiked with 100 nM of Ara h 1. FIG. 7 shows the relative increase in $R_{th}$ for respectively the baseline TGK buffer 71, the buffer with peanut extract 72 and the Ara h 1 spiked peanut extract in TGK buffer 73.

Upon exposing the functionalized gold sample to the peanut extract, a significant increase of 2.2 ± 0.8 % was observed. By using the dose-response curve in buffer solutions presented hereinabove as reference, this would correspond to an Ara h 1 concentration in the range of 1 to 3 nM, comparable to what was estimated with the procedure disclosed by Pomés et al., in Clin. Exp. All. 36, p. 824-830. When the spiked solution (100 nM Ara h 1) was introduced, the $R_{th}$ value went further up to 14 ± 0.7 %. This was slightly lower than what was obtained for 100 nM in buffer solutions; however, the presence of other components in such a complicated matrix may potentially hinder binding capacity of the aptamer. Nonetheless, the feasibility of allergen screening in food samples in accordance with embodiments of the present invention is clearly demonstrated by this example.

The results of these examples, obtained in accordance with embodiments of the present invention, were verified by QCM experiments. Three Au-coated QCM crystals were used: one having a pure gold surface, the second containing a SAM-layer treated with BSA, while a third was fully functionalized with SAM and aptamers and subsequently blocked with BSA. These references were used in order to rule out viscosity effects induced by the presence of the peanut butter and to compensate for the non-specific absorption since QCM crystals are mounted horizontally, allowing the heavy peanut allergen to sink to the surface.

This experiment was conducted according to the same procedure as for the examples in accordance with embodiments of the present invention, presented hereinabove; the signal was stabilized in TGK buffer after which first peanut extract was added followed by peanut extract spiked with 100 nM of Ara h 1. FIG. 8 shows the frequency shift obtained for respectively the baseline TGK buffer 81, the buffer with peanut extract 82 and the Ara h 1 spiked peanut extract in TGK buffer 83, obtained for respectively the first QCM crystal 84, which has a pure gold surface, the second QCM crystal 85, which has a BSA-treated SAM-layer and the third QCM crystal 86, which was functionalized with SAM and aptamers and blocked with BSA.

The addition of the peanut extract or the extract spiked with Ara h 1 did not result in a significant effect when the Au-coated QCM crystal was not functionalized. If the QCM crystals were treated with a SAM layer blocked with BSA, the crystal became more hydrophilic and was more prone to non-specific absorption. With only the peanut extract the increase was not significant yet but when spiked with 100 nM Ara h 1, an increase of 15 ± 1 Hz was measured. This was however still significantly lower than when the crystal was fully functionalized with aptamer; in that case with the extract already a significant increase of 6 ± 1 Hz was observed and with the spiked extract this resulted in 30 ± 1 Hz. By taking the difference between the reference experiment and the sample functionalized with aptamer, the signal solely attributed to specific binding of the allergen to the aptamer was determined to be 20 Hz. If validity of the Sauerbrey equation conditions is assumed, this frequency response corresponds to a mass difference of roughly 354 ng. While this method also enables the detection of spiked Ara h 1 in a complex matrix, the effect of the non-specific absorption is higher, which may be due to the different sample configuration, e.g. horizontal as opposed to vertical.

## Claims

**1.** A biosensor device (10) for detecting a predetermined target analyte, the biosensor device comprising:

- a substrate (13) comprising a bioreceptor (21) exposed at a functionalized surface thereof, said bioreceptor being adapted for specifically binding to the predetermined target analyte;
- a heat source (11) for heating said substrate (13) via a back surface thereof, said back surface being opposite of said functionalized surface and directed toward the heat source (11);
- a first temperature sensing element (14) for sensing a temperature at the side of the back surface of the substrate (13) and a second temperature sensing element (15) for sensing a temperature at the side of the functionalized surface of said substrate (13); and
- a signal processing unit (16) adapted for calculating at least one heat transfer resistivity value based on temperature values obtained from the first temperature sensing element and the second temperature sensing element and the heating power generated by the heat source (11),

**characterized in that** said bioreceptor (21) is an aptamer bioreceptor.

**2.** The biosensor device (10) according to claim 1, wherein said aptamer bioreceptor (21) is adapted for specifically binding to a predetermined target protein.

**3.** The biosensor device (10) according to claim 1, wherein said aptamer bioreceptor comprises a strand of synthetic oligonucleic acid or peptide domains.

**4.** The biosensor device (10) according to any of the previous claims, wherein said substrate (13) comprises a semiconductor substrate, a metal deposited on the surface of the semiconductor substrate, and a self-assembled monolayer (22) deposited on said metal, and wherein said aptamer bioreceptor (21) is chemically coupled to said self-assembled monolayer (22) such as to form said functionalized surface.

5. The biosensor device (10) according to any of the previous claims, wherein said aptamer bioreceptor (21) comprises a carbon spacer segment for spacing the aptamer bioreceptor away from the substrate (13).

6. The biosensor device (10) according to any of the previous claims, wherein said substrate (13) furthermore comprises a blocking layer (23) to reduce non-specific binding.

7. The biosensor device (10) according to any of the previous claims, wherein said heat source (11) comprises a solid body heat sink (19) and a heating element (20) for converting an input power to a caloric power.

8. The biosensor device (10) according to any of the previous claims, wherein said first temperature sensing element (14) is configured to measure a temperature of the solid body heat sink (19).

9. The biosensor device (10) according to claim 8, wherein the signal processing unit (16) comprises a proportional-integral derivative controller (18) for actively steering the temperature of the heat source (11), said proportional-integral derivative controller (18) being adapted for receiving a signal representative of said temperature of the solid body heat sink (19) as input and for outputting a signal to control the power of the heat source (11).

10. The biosensor device (10) according to any of the previous claims, wherein said first temperature sensing element (14) and/or said second temperature sensing element (15) comprise at least one thermocouple.

11. The biosensor device (10) according to any of the previous claims, wherein said substrate (13) is substantially horizontally arranged such that the functionalized surface is directed downward.

12. The biosensor device (10) according to any of the previous claims, furthermore comprising a flow cell (25) for bringing said functionalized surface of the substrate (13) into contact with a liquid sample (24), said flow cell (25) comprising a fluid compartment (28) for exposing the functionalized surface of the substrate (13) to the liquid sample (24).

13. The biosensor device (10) according to claim 12, wherein said flow cell (25) comprises a pumping and/or valve system for transferring fluid from and to the fluid compartment (28).

14. The biosensor device (10) according to claim 12 or claim 13, wherein said second temperature sensing element (15) is positioned in said fluid compartment (28).

15. A method for detecting a predetermined target analyte, the method comprising:

- obtaining a substrate (13) having a functionalized surface, the substrate comprising a bioreceptor (21) according to any of the claims 1-14 exposed at said functionalized surface, wherein said bioreceptor is adapted for specifically binding to the predetermined target analyte;
- providing a heating power for heating said substrate (13) at a back surface thereof, said back surface being opposite of said functionalized surface;
- sensing at least a temperature at a first side of the substrate corresponding to said back surface and at a second side of the substrate corresponding to said functionalized surface; and
- calculating at least one heat transfer resistivity value based on the temperature values obtained at the first side and the second side and the heating power for deriving a characteristic of the predetermined target analyte from said heat transfer resistivity value, **characterized in that** said bioreceptor (21) is an aptamer bioreceptor.

## Patentansprüche

1. Biosensorvorrichtung (10) zum Detektieren eines vorbestimmten Zielanalyten, wobei die Biosensorvorrichtung Folgendes umfasst:

- ein Substrat (13), das einen Biorezeptor (21) umfasst, der an einer funktionalisierten Oberfläche davon freiliegt, wobei der Biorezeptor angepasst ist, spezifisch an den vorbestimmten Zielanalyten zu binden;
- eine Wärmequelle (11) zum Erwärmen des Substrats (13) über eine Rückfläche davon, wobei die Rückfläche der funktionalisierten Oberfläche gegenüberliegt und zur Wärmequelle (11) gerichtet ist;

- einen ersten Temperaturfühler (14) zum Erfassen einer Temperatur auf der Seite der Rückfläche des Substrats (13) und einen zweiten Temperaturfühler (15) zum Erfassen einer Temperatur auf der Seite der funktionalisierten Oberfläche des Substrats (13); und
- eine Signalverarbeitungseinheit (16), die zum Berechnen von mindestens einem Wärmeübertragungswiderstandswert basierend auf Temperaturwerten, die von dem ersten Temperaturfühler und dem zweiten Temperaturfühler erhalten werden, und der von der Wärmequelle (11) erzeugten Heizleistung angepasst ist;

**dadurch gekennzeichnet, dass** der Biorezeptor (21) ein Aptamer-Biorezeptor ist.

**2.** Biosensorvorrichtung (10) nach Anspruch 1, wobei der Aptamer-Biorezeptor (21) zum spezifischen Binden an ein vorbestimmtes Zielprotein angepasst ist.

**3.** Biosensorvorrichtung (10) nach Anspruch 1, wobei der Aptamer-Biorezeptor einen Strang von synthetischen Oligonukleotidsäuren oder Peptiddomänen umfasst.

**4.** Biosensorvorrichtung (10) nach einem der vorstehenden Ansprüche, wobei das Substrat (13) ein Halbleitersubstrat, ein auf der Oberfläche des Halbleitersubstrats abgeschiedenes Metall und eine auf diesem Metall abgeschiedene selbstorganisierte Monoschicht (22) umfasst, und wobei der Aptamer-Biorezeptor (21) chemisch an die selbstorganisierte Monoschicht (22) gekoppelt ist, um die funktionalisierte Oberfläche zu bilden.

**5.** Biosensorvorrichtung (10) nach einem der vorstehenden Ansprüche, wobei der Aptamer-Biorezeptor (21) ein Kohlenstoff-Abstandshaltersegment zum Beabstanden des Aptamer-Biorezeptors von dem Substrat (13) umfasst.

**6.** Biosensorvorrichtung (10) nach einem der vorstehenden Ansprüche, wobei das Substrat (13) ferner eine Blockierungsschicht (23) umfasst, um eine unspezifische Bindung zu reduzieren.

**7.** Biosensorvorrichtung (10) nach einem der vorstehenden Ansprüche, wobei die Wärmequelle (11) eine Festkörper-Wärmesenke (19) und ein Heizelement (20) zum Umwandeln einer Eingangsleistung in eine Kalorienleistung umfasst.

**8.** Biosensorvorrichtung (10) nach einem der vorstehenden Ansprüche, wobei der erste Temperaturfühler (14) konfiguriert ist, um eine Temperatur der Feststoffkörperwärmesenke (19) zu messen.

**9.** Biosensorvorrichtung (10) nach Anspruch 8, wobei die Signalverarbeitungseinheit (16) einen Proportional-Integral-Differential-Regler (18) zum aktiven Steuern der Temperatur der Wärmequelle (11) umfasst, wobei der Proportional-Integral-Differential-Regler (18) dazu angepasst ist, ein für die Temperatur der Festkörper-Wärmesenke (19) repräsentatives Signal als Eingang zu empfangen und ein Signal zur Steuerung der Leistung der Wärmequelle (11) abzugeben.

**10.** Biosensorvorrichtung (10) nach einem der vorstehenden Ansprüche, wobei der erste Temperaturfühler (14) und / oder der zweite Temperaturfühler (15) mindestens ein Thermoelement umfassen.

**11.** Biosensorvorrichtung (10) nach einem der vorstehenden Ansprüche, wobei das Substrat (13) im Wesentlichen horizontal angeordnet ist, sodass die funktionalisierte Oberfläche nach unten gerichtet ist.

**12.** Biosensorvorrichtung (10) nach einem der vorstehenden Ansprüche, ferner umfassend eine Durchflusszelle (25) zum Inkontaktbringen der funktionalisierten Oberfläche des Substrats (13) mit einer flüssigen Probe (24), wobei die Durchflusszelle (25) eine Fluidkammer (28) umfasst, um die funktionalisierte Oberfläche des Substrats (13) der flüssigen Probe (24) auszusetzen.

**13.** Biosensorvorrichtung (10) nach Anspruch 12, wobei die Durchflusszelle (25) ein Pump und / oder Ventilsystem zum Übertragen von Fluid von und zu der Fluidkammer (28) umfasst.

**14.** Biosensorvorrichtung (10) nach Anspruch 12 oder Anspruch 13, wobei der zweite Temperaturfühler (15) in der Fluidkammer (28) positioniert ist.

**15.** Verfahren zum Nachweisen eines vorbestimmten Zielanalyten, wobei das Verfahren umfasst:

- Erhalten eines Substrats (13), das eine funktionalisierte Oberfläche aufweist, wobei das Substrat einen Biorezeptor (21) nach einem der Ansprüche 1 bis 14 umfasst, der an der funktionalisierten Oberfläche freiliegt, wobei der Biorezeptor zur spezifischen Bindung an den vorbestimmten Zielanalyten angepasst ist;
- Bereitstellen einer Heizleistung zum Heizen des Substrats (13) an einer Rückfläche davon, wobei die Rückfläche der funktionalisierten Oberfläche gegenüberliegt;
- Erfassen mindestens einer Temperatur auf einer ersten Seite des Substrats entsprechend der hinteren Oberfläche und auf einer zweiten Seite des Substrats entsprechend der funktionalisierten Oberfläche; und
- Berechnen mindestens eines Wärmeübertragungswiderstandswerts basierend auf den Temperaturwerten, die auf der ersten Seite und der zweiten Seite erhalten werden, und der Heizleistung zum Ableiten einer Eigenschaft des vorbestimmten Zielanalyten aus dem Wärmeübertragungswiderstandswert, **dadurch gekennzeichnet, dass** der Biorezeptor (21) ein Aptamer-Biorezeptor ist.

## Revendications

**1.** Dispositif de biodétection (10) pour détecter un analyte cible prédéterminé, le dispositif de biodétection comprenant :

- un substrat (13) comprenant un biorécepteur (21) exposé sur une surface fonctionnalisée de celui-ci, ledit biorécepteur étant adapté pour se lier spécifiquement à l'analyte cible prédéterminé ;
- une source de chaleur (11) pour chauffer ledit substrat (13) via une surface arrière de celui-ci, ladite surface arrière étant opposée à ladite surface fonctionnalisée et dirigée vers la source de chaleur (11) ;
- un premier élément de détection de la température (14) pour détecter une température du côté de la surface arrière du substrat (13) et un deuxième élément de détection de la température (15) pour détecter une température du côté de la surface fonctionnalisée dudit substrat (13) ; et
- une unité de traitement de signal (16) adaptée pour calculer au moins une valeur de résistivité de transfert de chaleur sur la base des valeurs de température obtenues à partir du premier élément de détection de la température et du deuxième élément de détection de la température et de la puissance thermique générée par la source de chaleur (11),

**caractérisé en ce que** ledit biorécepteur (21) est un biorécepteur aptamère.

**2.** Dispositif de biodétection (10) selon la revendication 1, dans lequel ledit biorécepteur aptamère (21) est adapté pour se lier spécifiquement à une protéine cible prédéterminée.

**3.** Dispositif de biodétection (10) selon la revendication 1, dans lequel ledit biorécepteur aptamère comprend un brin d'acide oligonucléique synthétique ou des domaines peptidiques.

**4.** Dispositif de biodétection (10) selon l'une quelconque des revendications précédentes, dans lequel ledit substrat (13) comprend un substrat semi-conducteur, un métal déposé sur la surface du substrat semi-conducteur, et une monocouche auto-assemblée (22) déposée sur ledit métal, et dans lequel ledit biorécepteur aptamère (21) est couplé chimiquement à ladite monocouche auto-assemblée (22) de façon à former ladite surface fonctionnalisée.

**5.** Dispositif de biodétection (10) selon l'une quelconque des revendications précédentes, dans lequel ledit biorécepteur aptamère (21) comprend un segment d'espacement carbone pour espacer le biorécepteur aptamère du substrat (13).

**6.** Dispositif de biodétection (10) selon l'une quelconque des revendications précédentes, dans lequel ledit substrat (13) comprend en outre une couche de blocage (23) pour réduire la liaison non spécifique.

**7.** Dispositif de biodétection (10) selon l'une quelconque des revendications précédentes, dans lequel ladite source de chaleur (11) comprend un dissipateur thermique à corps solide (19) et un élément chauffant (20) pour convertir une puissance d'entrée en une puissance calorique.

**8.** Dispositif de biodétection (10) selon l'une quelconque des revendications précédentes, dans lequel ledit premier élément de détection de la température (14) est configuré pour mesurer une température du dissipateur thermique à corps solide.

**9.** Dispositif de biodétection (10) selon la revendication 8, dans lequel l'unité de traitement de signal (16) comprend un régulateur proportionnel intégral et dérivé (18) pour commander activement la température de la source de chaleur (11), ledit régulateur proportionnel intégral et dérivé (18) étant adapté pour recevoir un signal représentatif de ladite température du dissipateur thermique à corps solide (19) comme entrée et pour émettre un signal pour commander la puissance de la source de chaleur (11).

**10.** Dispositif de biodétection (10) selon l'une quelconque des revendications précédentes, dans lequel ledit premier élément de détection de la température (14) et/ou ledit deuxième élément de détection de la température (15) comprend au moins un thermocouple.

**11.** Dispositif de biodétection (10) selon l'une quelconque des revendications précédentes, dans lequel ledit substrat (13) est sensiblement agencé horizontalement de sorte que la surface fonctionnalisée soit directement vers le bas.

**12.** Dispositif de biodétection (10) selon l'une quelconque des revendications précédentes, comprenant en outre une cellule d'écoulement (25) pour mettre en contact ladite surface fonctionnalisée du substrat (13) avec un échantillon liquide (24), ladite cellule d'écoulement (25) comprenant un compartiment de fluide (28) pour exposer la surface fonctionnalisée du substrat (13) à l'échantillon liquide (24).

**13.** Dispositif de biodétection (10) selon la revendication 12, dans lequel ladite cellule d'écoulement (25) comprend un système de pompage et/ou un système de vannes pour transférer du fluide depuis et jusqu'au compartiment de fluide (28).

**14.** Dispositif de biodétection (10) selon la revendication 12 ou la revendication 13, dans lequel ledit deuxième élément de détection de la température (15) est positionné dans ledit compartiment de fluide (28).

**15.** Procédé de détection d'un analyte cible prédéterminé, le procédé comprenant :

- l'obtention d'un substrat (13) ayant une surface fonctionnalisée, le substrat comprenant un biorécepteur (21) selon l'une quelconque des revendications 1 à 14 exposé sur ladite surface fonctionnalisée, dans lequel ledit biorécepteur est adapté pour se lier spécifiquement à l'analyte cible prédéterminé ;
- la fourniture d'une puissance thermique pour chauffer ledit substrat (13) sur une surface arrière de celui-ci, ladite surface arrière étant opposée à ladite surface fonctionnalisée ;
- la détection d'au moins une température sur un premier côté du substrat correspondant à ladite surface arrière et sur un deuxième côté du substrat correspondant à ladite surface fonctionnalisée ; et
- le calcul d'au moins une valeur de résistivité de transfert de chaleur sur la base des valeurs de température obtenues sur le premier côté et le deuxième côté et la puissance de chauffage pour dériver une caractéristique de l'analyte cible prédéterminé à partir de ladite valeur de résistivité de transfert thermique, **caractérisé en ce que** ledit biorécepteur (21) est un biorécepteur aptamère.

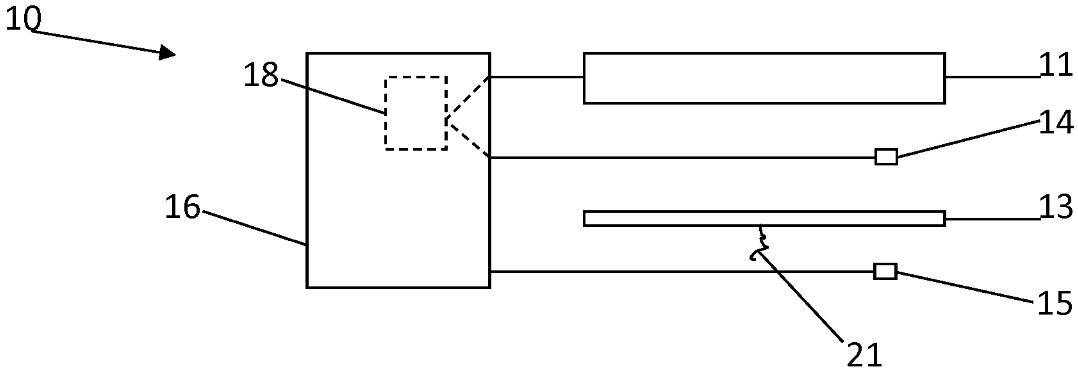


FIG. 1

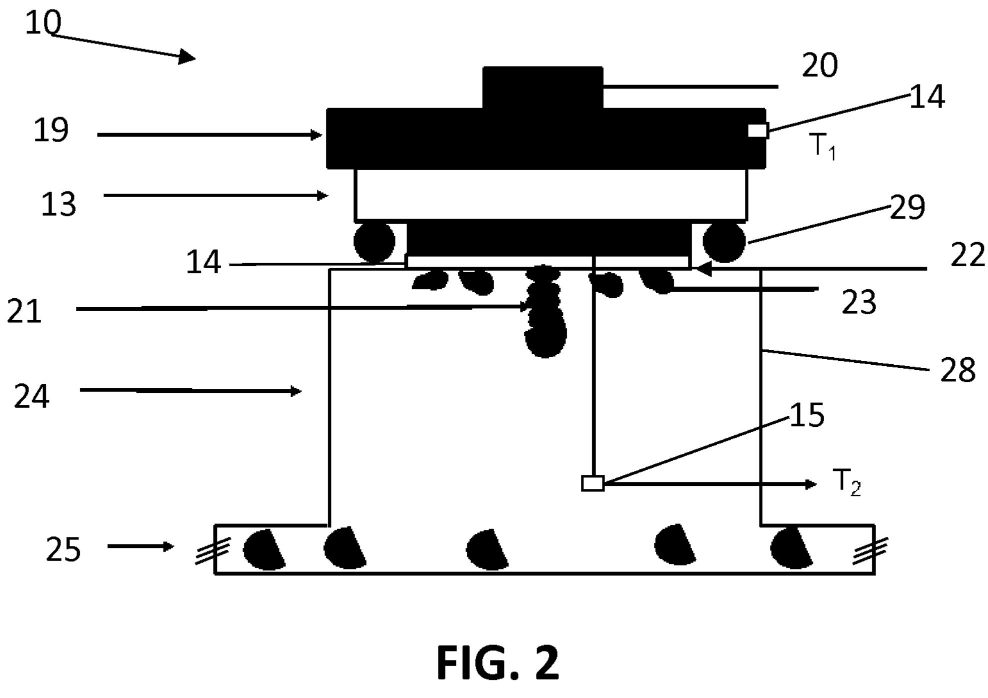


FIG. 2

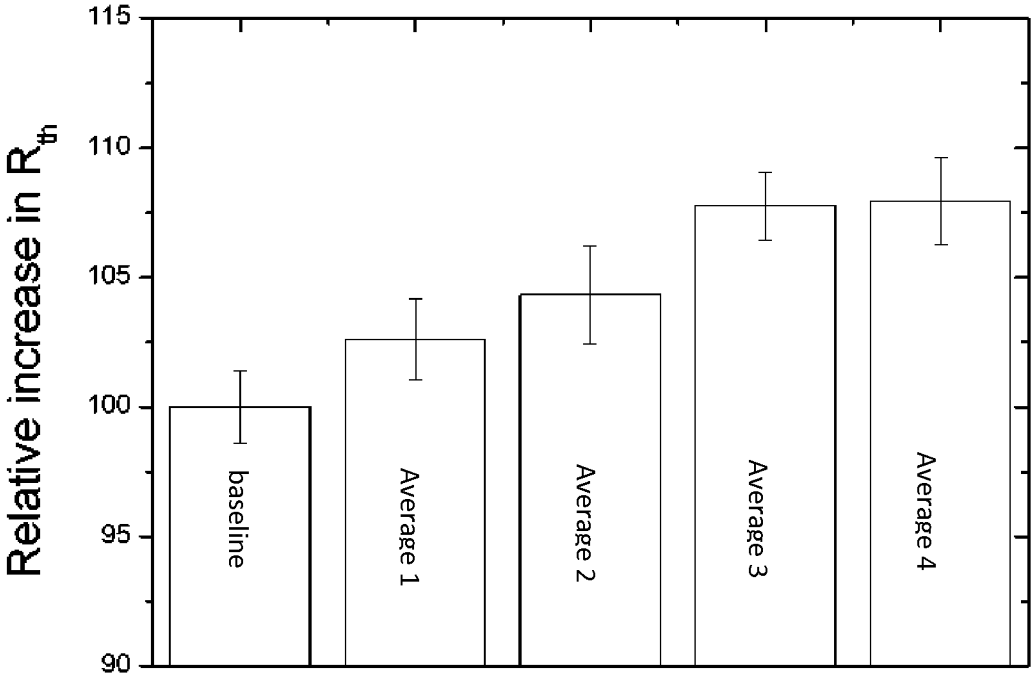
115
110
105
100
95
90
Relative increase in $R_{th}$
baseline
Average 1
Average 2
Average 3
Average 4

FIG. 3

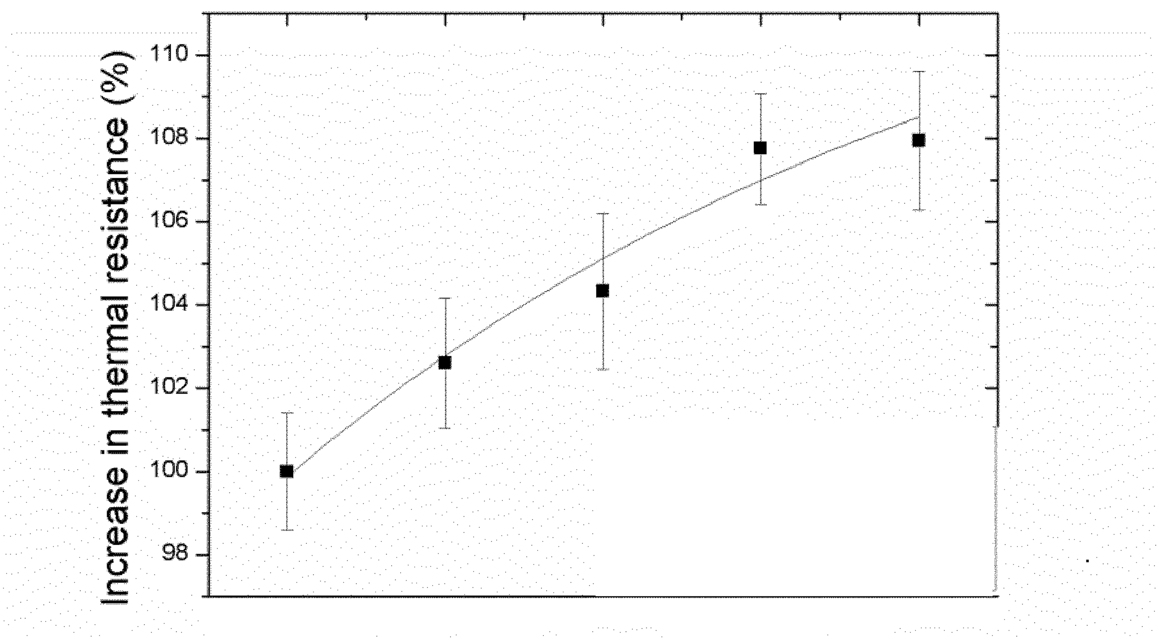
110
108
106
104
102
100
98
Increase in thermal resistance (%)

FIG. 4

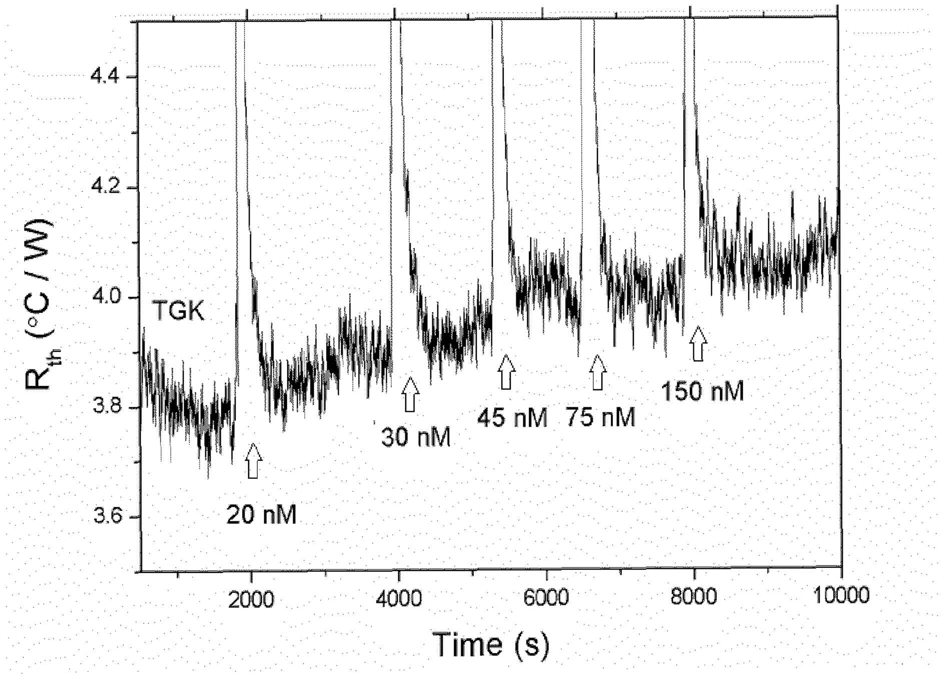

4.4
4.2
4.0
3.8
3.6
$R_{th}$ (°C / W)
TGK
20 nM
30 nM
45 nM
75 nM
150 nM
2000
4000
6000
8000
10000
Time (s)


FIG. 5

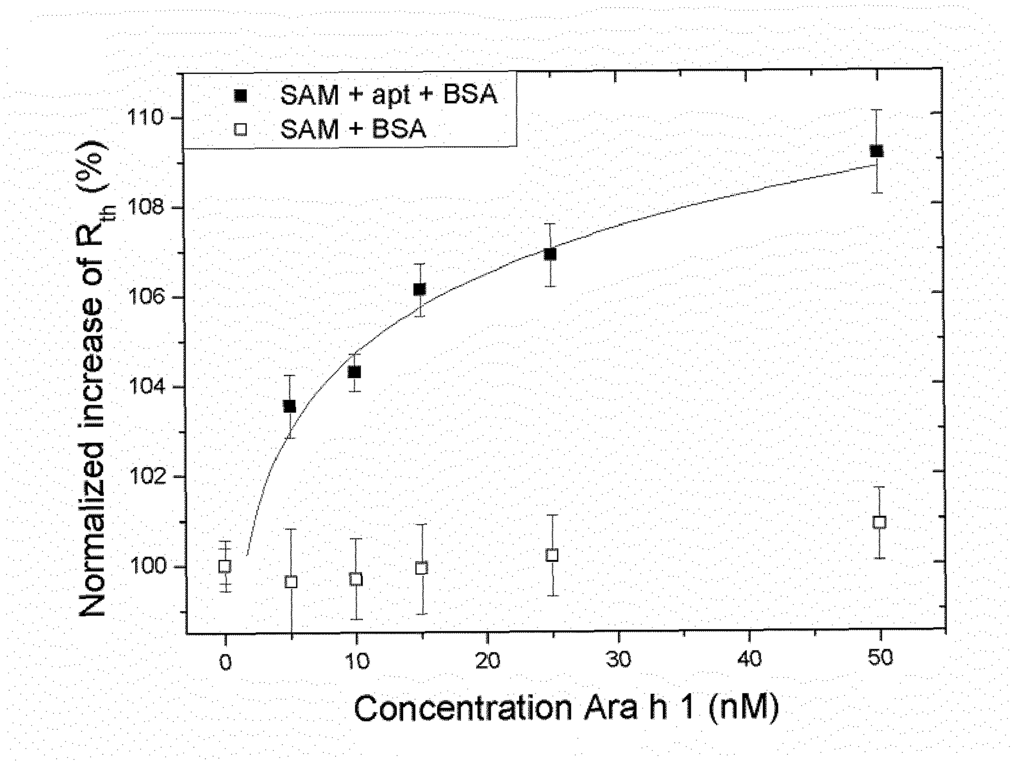

SAM + apt + BSA
SAM + BSA
110
108
106
104
102
100
Normalized increase of $R_{th}$ (%)
0
10
20
30
40
50
Concentration Ara h 1 (nM)


FIG. 6

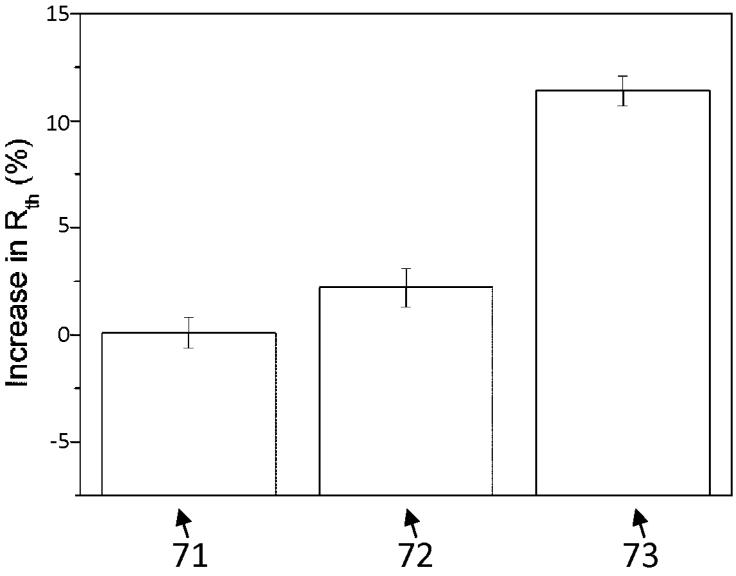
15
10
5
0
-5
Increase in $R_{th}$ (%)
71
72
73

FIG. 7

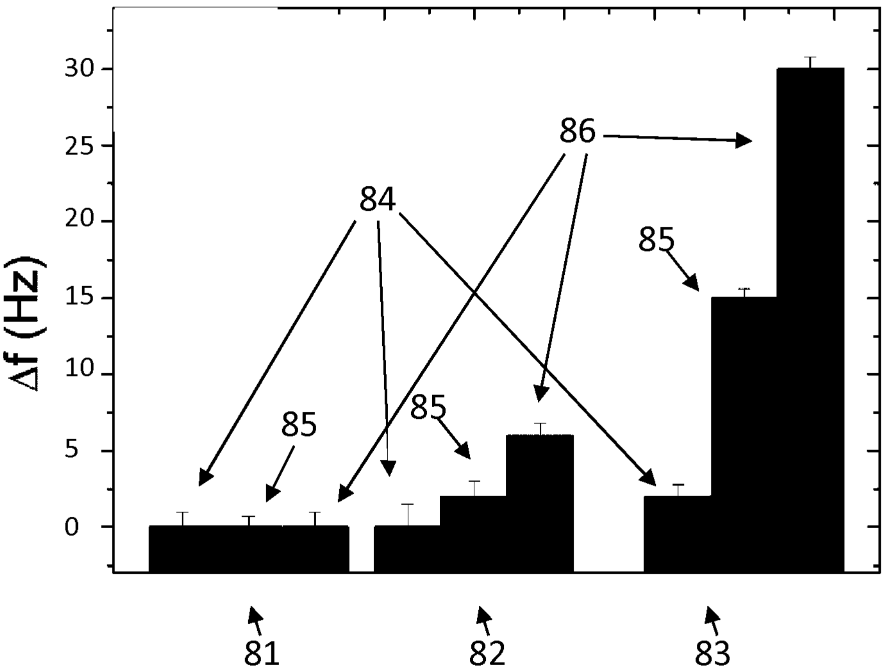
30
25
20
15
10
5
0
Δf (Hz)
86
84
85
85
85
81
82
83

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **PEETERS et al.** *J. Biosens. Bioelectron.,* 5
- **PEETERS.** Heat-transfer-based detection of 1-nicotine, histamine, and serotonin using molecularly imprinted polymers as biomimetic receptors. *Analytical and bioanalytical chemistry,* vol. 405 (20), 6453-6460
- **GEERETS.** Optimizing the thermal read-out technique for MIP-based biomimetic sensors: towards nanomolar detection limits. *Sensors,* vol. 13 (7), 9148-9159
- **ZHU.** Specific capture and temperature-mediated release of cells in an aptamer-based microfluidic device. *Lab on a chip,* vol. 12 (18), 3504
- **WACKERS.** Array formatting of the heat-transfer method (HTM) for the detection of small organic molecules by molecularly imprinted polymers. *Sensors,* vol. 14 (6), 11016-11030
- **TRAN et al.** *Biosens. Bioelectron.,* vol. 43, 245-251
- **POMÉS et al.** *Clin. Exp. All.,* vol. 36, 824-830